# EUROPEAN PATENT APPLICATION

(11) **EP 4 744 666 A1**
(43) Date of publication of application: **20.05.2026**
(21) Application number: 24842302.2
(22) Date of filing: 12.07.2024
(51) Int. Cl.: A61K 31/551, C07D 487/14, C07D 519/00, A61P 35/00, A61P 35/04

(54) **PHARMACEUTICAL COMPOSITION AND USE THEREOF**

(30) Priority: 14.07.2023 CN 202310866688; 06.09.2023 CN 202311144318; 28.09.2023 CN 202311273731; 25.05.2024 CN 202410657348
(71) Applicant: TransThera Sciences (Nanjing), Inc., Nanjing, Jiangsu 210032 (CN)
(72) Inventor: PENG, Peng, Nanjing, Jiangsu 210032 (CN); QIANG, Xiaoyan, Nanjing, Jiangsu 210032 (CN); ZENG, Yucheng, Nanjing, Jiangsu 210032 (CN); FAN, Jing, Gaithersburg, Maryland 20878-5789 (US)
(74) Representative: Winter, Brandl - Partnerschaft mbB
(86) International application number: PCT/CN2024/105173
(87) International publication number: WO 2025/016309

(57) **Abstract**

A pharmaceutical composition and a use thereof, specifically relating to (a) a compound of general formula (I) or a pharmaceutically acceptable salt, isomer or crystal form thereof, and (b) a novel endocrine therapy drug, variables in the general formula each being as defined in the description. It has been found by means of research that the compound of general formula (I) or the pharmaceutically acceptable salt, isomer or crystal form thereof, and the novel endocrine therapy drug, have a synergistic effect in combined treatment of prostate cancer.

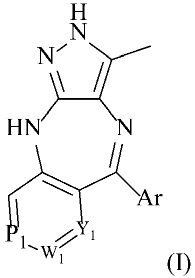

## Description

### TECHNICAL FIELD

The present disclosure pertains to the technical field of pharmaceuticals, and particularly relates to a pharmaceutical composition and use thereof.

### BACKGROUND

Prostate cancer (PCa) is one of the most common malignancies in men. Globally, PCa ranks second in incidence among male tumors, and ranks first in incidence among male malignancies in the United States, with a mortality rate second only to lung cancer.

Based on the staging and risk level of prostate cancer, treatment regimens vary accordingly. For patients with early-stage, low-risk prostate cancer, active surveillance is mainly adopted. Due to reasons such as the absence of obvious symptoms or limitations in detection means for early-stage prostate cancer, the majority of prostate cancer cases are diagnosed at an advanced stage, with half or more having already developed distant metastasis. The 5-year relative survival rate for those with distant metastasis is only 30%. For patients with advanced, metastatic prostate cancer, the main treatment means is medical or surgical castration, namely androgen deprivation therapy (ADT). Because the growth of both normal prostate cells and prostate cancer cells is androgen-dependent, the concentration of androgens in a patient's body can be minimized by surgical or medical castration, thereby inhibiting the androgen signaling pathway and ultimately inhibiting the growth of tumor cells. Therapeutic drugs currently used for ADT include gonadotropin-releasing hormone agonists (GnRH agonists), gonadotropin-releasing hormone antagonists (GnRH antagonists), androgen secretion inhibitors, and the like. Most patients are initially sensitive to ADT, meaning they have castration-sensitive prostate cancer (CSPC). However, due to reasons such as genetics or drug induction, almost all patients will progress to castration-resistant prostate cancer (CRPC) after 2 years, developing drug resistance. Once the cancer progresses to CRPC, the median survival time for the patients is only 9-12 months.

CRPC mainly has two independent stages: metastatic castration-resistant prostate cancer (mCRPC) with distant metastasis and non-metastatic castration-resistant prostate cancer (nmCRPC) without distant metastasis. When patients with nmCRPC experience distant metastasis or disease progression after failure of novel hormone therapy, they transition to the mCRPC stage, so mCRPC can be considered the final stage for patients with PCa. The main therapies currently used for mCRPC include chemotherapy (docetaxel), novel hormone therapy (enzalutamide, abiraterone acetate, etc.), immunotherapy (Sipuleucel-T), and radiotherapy for bone metastases (radium-223). Docetaxel is the first chemotherapy drug approved by the FDA for the treatment of mCRPC, capable of effectively extending the overall survival (OS) of patients by about 19.2 months. However, this drug causes a series of side effects, such as nausea, vomiting, neurological disorders, anemia, etc. 17α-hydroxylase/C17,20-lyase (CPY17 enzyme) plays a key role in the process of androgen synthesis. Abiraterone acetate is a prodrug that is converted into abiraterone *in vivo,* and abiraterone is an androgen synthesis inhibitor that can lower androgen levels by inhibiting the CYP17 enzyme. This drug was approved by the FDA in 2011 for use in patients with CRPC. Enzalutamide is a second-generation AR (androgen receptor) inhibitor. After AR binds to enzalutamide, it cannot be normally transported into the cell nucleus or recruit coactivators, resulting in reduced AR transcriptional activity. Compared with first-generation AR inhibitors, enzalutamide has a stronger affinity for AR. Although the approval of drugs such as abiraterone acetate and enzalutamide is of great significance for the treatment of mCRPC, most patients ultimately develop drug resistance after a period of treatment.

Due to the issues of tolerability and safety of therapeutic drugs, the treatment of prostate cancer remains a clinical challenge, and exploring effective treatment methods is urgently needed.

### SUMMARY

In order to solve the problems described above, the present disclosure provides the following solutions:
In a first aspect, the present disclosure provides a pharmaceutical composition, which comprises a component (a) and a component (b):
(a) a compound of general formula (I) or a pharmaceutically acceptable salt, an isomer, or a crystal form thereof, and
(b) a novel hormone therapy drug; 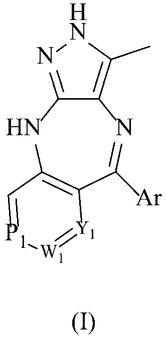
   wherein Ar is phenyl optionally substituted with 1-3 R⁶, each R⁶ being independently selected from hydrogen, amino, cyano, halogen, C₁₋₄ alkyl, and trifluoromethyl;
   Y₁ is CR³;
   P₁ is CR⁴;
   W₁ is N;
   R³ is hydrogen or C₁₋₄ alkyl;
   R⁴ is -(CH₂)ₙ-(5-11) membered heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl.

In some embodiments, Ar is phenyl optionally substituted with 1-3 R⁶, each R⁶ being independently selected from hydrogen and halogen;
Y₁ is CR³, and R³ is hydrogen;
P₁ is CR⁴, and R⁴ is selected from -(CH₂)ₙ-(5-6) membered monocyclic heterocyclyl and -(CH₂)ₙ-(7-11) membered polycyclic heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
W₁ is N;
preferably, the weight ratio of the component (a) to the component (b) is 1:2000 to 1000:2000, for example, 10:2000-1000:2000, or 100:2000-1000:2000, including but not limited to: 10:2000, 12:2000, 13.3:2000, 16:2000, 20:2000, 21.3:2000, 24:2000, 26.7:2000, 32:2000, 40:2000, 62.5:2000, 75:2000, 83.3:2000, 100:2000, 125:2000, 133.3:2000, 150:2000, 166.7:2000, 200:2000, and 250:2000.

In a second aspect, the present disclosure provides a pharmaceutical combination, which comprises or consists of a component (a) and a component (b), wherein the component (a) and the component (b) serve as active ingredients:
(a) a compound of general formula (I) or a pharmaceutically acceptable salt, an isomer, or a crystal form thereof, and
(b) a novel hormone therapy drug; 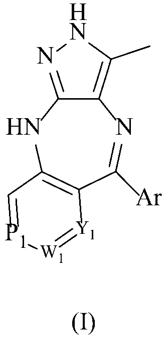
   wherein Ar is phenyl optionally substituted with 1-3 R⁶, each R⁶ being independently selected from hydrogen, amino, cyano, halogen, C₁₋₄ alkyl, and trifluoromethyl;
   Y₁ is CR³;
   P₁ is CR⁴;
   W₁ is N;
   R³ is hydrogen or C₁₋₄ alkyl;
   R⁴ is -(CH₂)ₙ-(5-11) membered heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl.

In some embodiments, Ar is phenyl optionally substituted with 1-3 R⁶, each R⁶ being independently selected from hydrogen and halogen;
Y₁ is CR³, and R³ is hydrogen;
P₁ is CR⁴, and R⁴ is selected from -(CH₂)ₙ-(5-6) membered monocyclic heterocyclyl and -(CH₂)ₙ-(7-11) membered polycyclic heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
W₁ is N;
preferably, the weight ratio of the component (a) to the component (b) is 1:2000 to 1000:2000, for example, 10:2000-1000:2000, or 100:2000-1000:2000, including but not limited to: 10:2000, 12:2000, 13.3:2000, 16:2000, 20:2000, 21.3:2000, 24:2000, 26.7:2000, 32:2000, 40:2000, 62.5:2000, 75:2000, 83.3:2000, 100:2000, 125:2000, 133.3:2000, 150:2000, 166.7:2000, 200:2000, and 250:2000.

In any embodiment of the pharmaceutical combination and the pharmaceutical composition described above, the (5-6) membered monocyclic heterocyclyl is (5-6) membered saturated monocyclic heterocyclyl, and the (7-11) membered polycyclic heterocyclyl is (7-11) membered saturated polycyclic heterocyclyl. In a preferred embodiment, the (7-11) membered saturated polycyclic heterocyclyl is (7-11) membered saturated ortho-fused heterocyclyl, (7-11) membered saturated spiro-heterocyclyl, or (7-11) membered saturated bridged heterocyclyl.

In any embodiment of the pharmaceutical combination and the pharmaceutical composition described above, R⁴ is 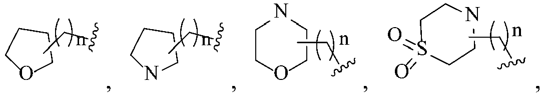 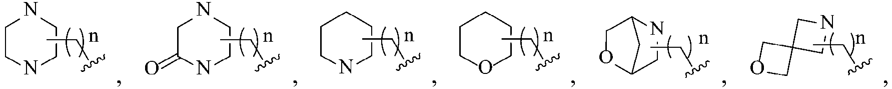 or 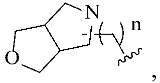 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

In any embodiment of the pharmaceutical combination and the pharmaceutical composition described above, R⁴ is 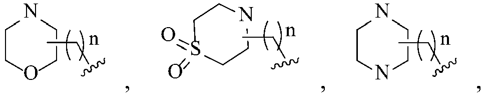 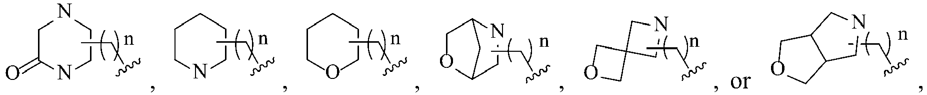 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

In any embodiment of the pharmaceutical combination and the pharmaceutical composition described above, R⁴ is 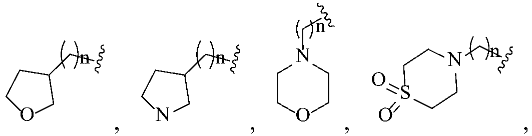 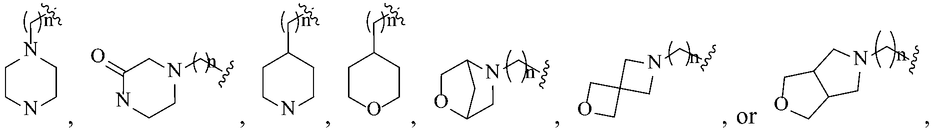 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl).

In any embodiment of the pharmaceutical combination and the pharmaceutical composition described above, R⁴ is 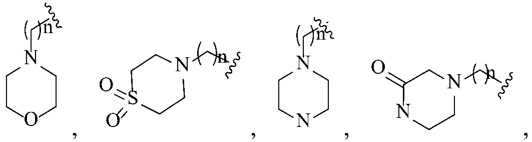 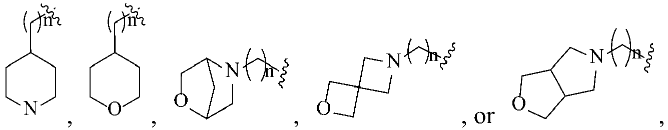 and n = an integer of 0-3, wherein the heterocyclyl in R⁴ is optionally substituted with one or more substituents independently selected from C₁₋₃ alkyl and C₃₋₆ cycloalkyl.

In any embodiment of the pharmaceutical combination and the pharmaceutical composition described above, the compound of general formula (I) includes the compounds shown in Table 1.

**Table 1: Examples of the compound of general formula (I)**

| No. | Structure | No. | Structure |
|---|---|---|---|
| 22 | 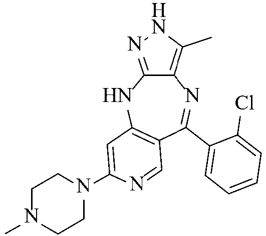 | 29 | 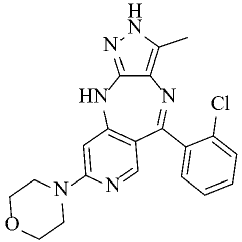 |
| 33 | 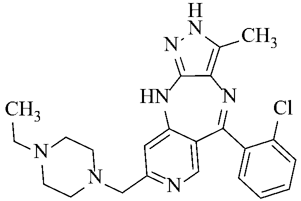 | 34 | 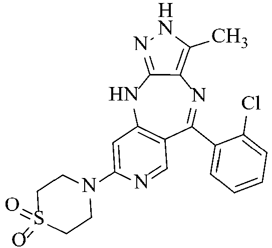 |
| 35 | 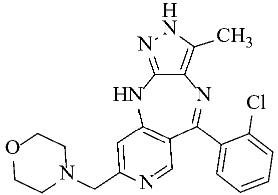 | 36 | 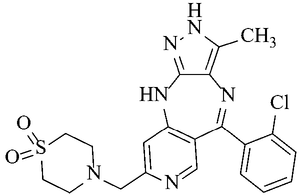 |
| 37 | 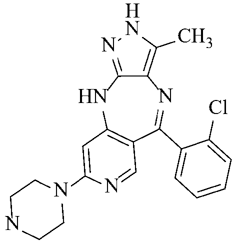 | 38 | 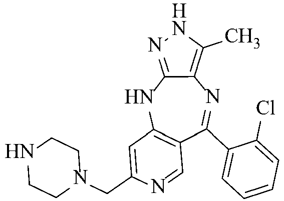 |
| 39 | 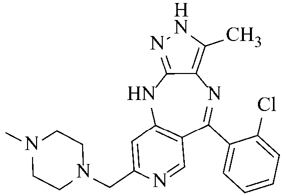 | 40 | 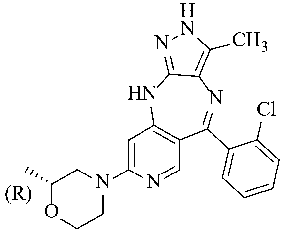 |
| 41 | 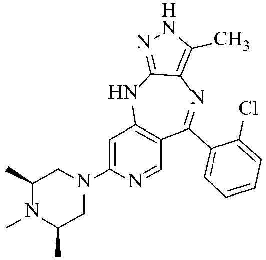 | 42 | 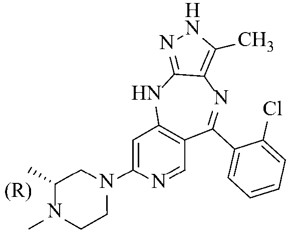 |
| 43 | 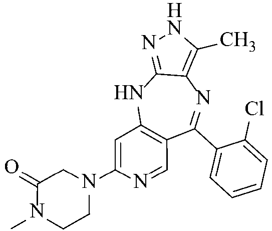 | 44 | 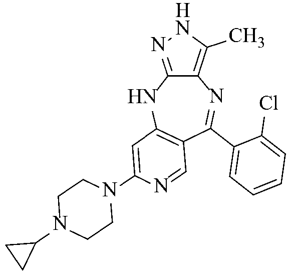 |
| 45 | 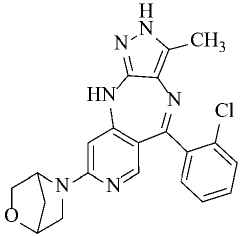 | 46 | 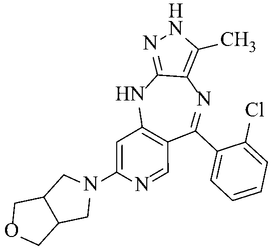 |
| 47 | 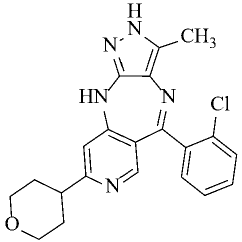 | 48 | 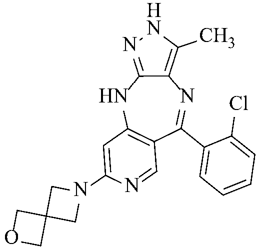 |
| 49 | 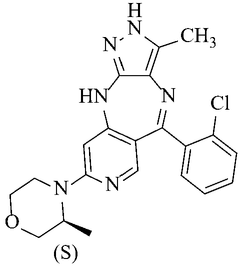 | 50 | 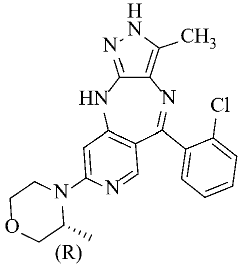 |
| 51 | 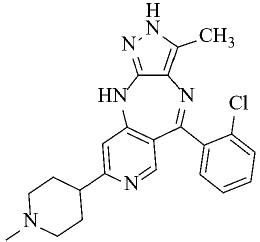 | | |

Preparation examples of the compounds in the above table, as well as examples verifying the biological activity of these compounds, can be found in the patent application WO2018108079A1, which is incorporated herein by reference in its entirety.

In any embodiment of the pharmaceutical combination and the pharmaceutical composition described above, the compound of the present disclosure may also include compounds other than the compound of general formula (I), for example, the specific compounds shown in the table below.

| No. | Structure | No. | Structure |
|---|---|---|---|
| 1 | 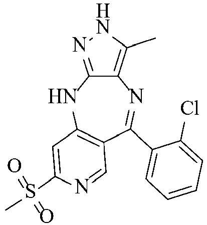 | 2 | 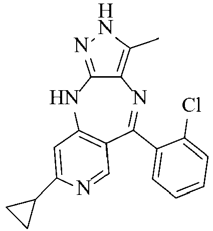 |
| 3 | 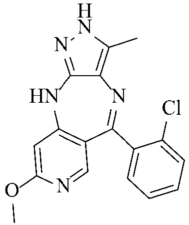 | 4 | 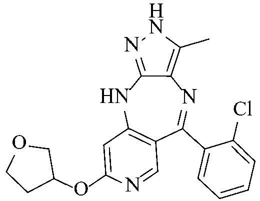 |
| 5 | 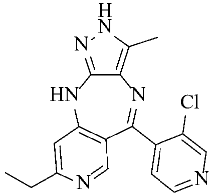 | 6 | 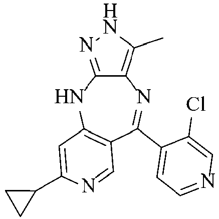 |
| 7 | 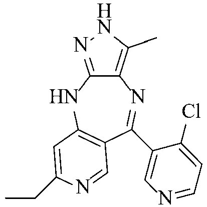 | 8 | 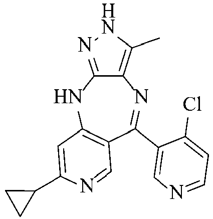 |
| 9 | 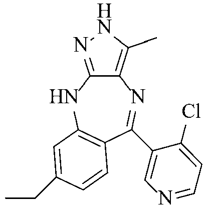 | 10 | 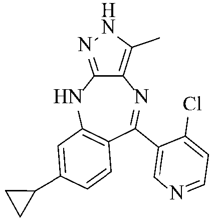 |
| 11 | 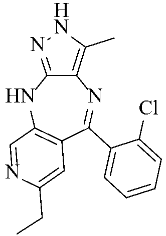 | 12 | 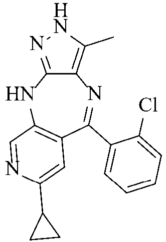 |
| 13 | 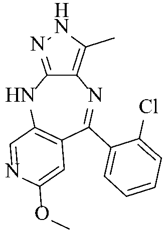 | 14 | 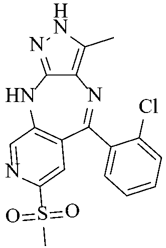 |
| 15 | 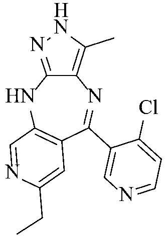 | 16 | 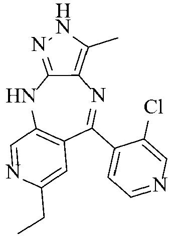 |
| 17 | 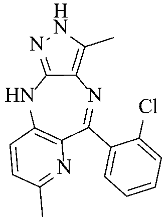 | 18 | 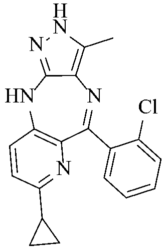 |
| 19 | 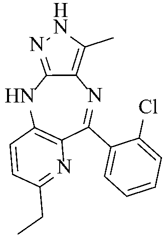 | 20 | 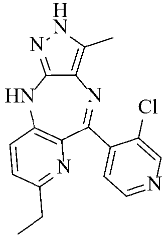 |
| 21 | 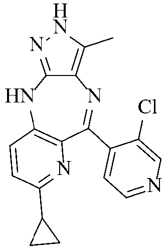 | 30 | 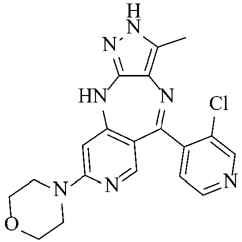 |
| 23 | 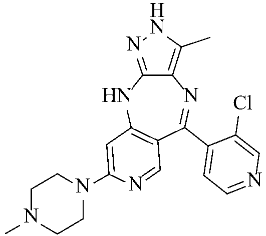 | 24 | 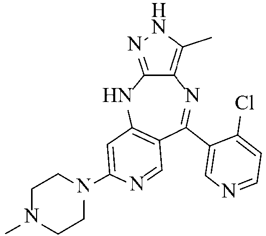 |
| 25 | 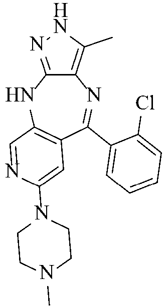 | 26 | 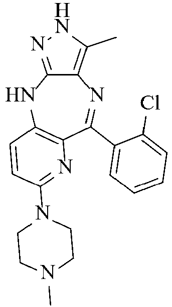 |
| 27 | 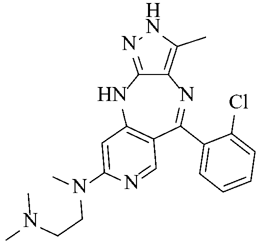 | 28 | 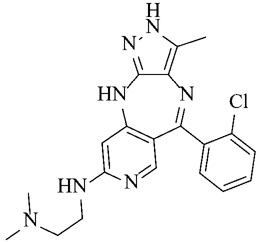 |
| 31 | 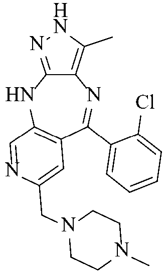 | 32 | 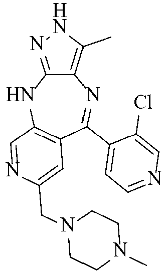 |

Preparation examples of the compounds in the above table, as well as examples verifying the biological activity of these compounds, can be found in the patent application WO2018108079A1, which is incorporated herein by reference in its entirety.

In any embodiment of the pharmaceutical combination and the pharmaceutical composition described above, the novel hormone therapy drug is an androgen receptor inhibitor.

In any embodiment of the pharmaceutical combination and the pharmaceutical composition described above, the androgen receptor inhibitor is enzalutamide, apalutamide, darolutamide, or bicalutamide.

In any embodiment of the pharmaceutical combination and the pharmaceutical composition described above, the androgen receptor inhibitor is rezvilutamide.

In any embodiment of the pharmaceutical combination and the pharmaceutical composition described above, the androgen receptor inhibitor is enzalutamide.

In any embodiment of the pharmaceutical combination and the pharmaceutical composition described above, the (a) is 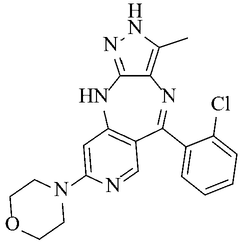 or a pharmaceutically acceptable salt, an isomer, or a crystal form thereof; the (b) novel hormone therapy drug is enzalutamide.

In any embodiment of the pharmaceutical combination and the pharmaceutical composition described above, the novel hormone therapy drug is an androgen synthesis inhibitor.

In any embodiment of the pharmaceutical combination and the pharmaceutical composition described above, the androgen synthesis inhibitor is abiraterone or abiraterone acetate.

In any embodiment of the pharmaceutical combination and the pharmaceutical composition described above, the (a) is 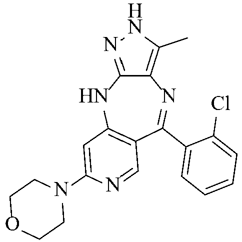 or a pharmaceutically acceptable salt, an isomer, or a crystal form thereof; the (b) novel hormone therapy drug is abiraterone or abiraterone acetate.

In any one of the above embodiments of the pharmaceutical combination and the pharmaceutical composition, the component (a) and the component (b) are provided in the form of one inseparable entity, or the component (a) and the component (b) are provided separately in different entities.

In a third aspect, the present disclosure further provides a kit, which comprises the pharmaceutical combination or the pharmaceutical composition according to any one of the preceding embodiments, and instructions for administration.

Further, the kit of the present disclosure comprises:
a) one or more unit formulations of the compound or the pharmaceutically acceptable salt, the isomer, or the crystal form thereof according to the first or second aspect of the present disclosure, i.e., the component (a);
b) one or more unit formulations of the novel hormone therapy drug according to the first or second aspect of the present disclosure, i.e., the component (b); and
c) instructions for administration.

Preferably, the a) one or more unit formulations of the compound or the pharmaceutically acceptable salt, the isomer, or the crystal form thereof according to the first or second aspect of the present disclosure and the b) one or more unit formulations of the novel hormone therapy drug according to the first or second aspect of the present disclosure are provided in the form of one inseparable entity, or provided separately in different entities.

In a fourth aspect, the present disclosure further provides a method of combination therapy for treating prostate cancer, which comprises administering to a prostate cancer patient in need thereof an effective amount of a pharmaceutical combination or a pharmaceutical composition or a kit comprising the compound or the pharmaceutically acceptable salt, the isomer, or the crystal form thereof according to the first or second aspect of the present disclosure, and the novel hormone therapy drug according to the first or second aspect of the present disclosure.

In a fifth aspect, the present disclosure further provides a pharmaceutical combination or a pharmaceutical composition or a kit comprising the compound or the pharmaceutically acceptable salt, the isomer, or the crystal form thereof according to the first or second aspect of the present disclosure, and the novel hormone therapy drug according to the first or second aspect of the present disclosure, for use in preventing and/or treating prostate cancer.

In a sixth aspect, the present disclosure further provides use of the pharmaceutical combination or the pharmaceutical composition or the kit according to any one of the first, second, and third aspects described above in the manufacture of a medicament for preventing and/or treating prostate cancer.

In any embodiment of the sixth aspect, the use further comprises that: the compound or the pharmaceutically acceptable salt, the isomer, or the crystal form thereof according to the first or second aspect of the present disclosure, and the novel hormone therapy drug according to the first or second aspect of the present disclosure in the pharmaceutical combination or the pharmaceutical composition or the kit are provided separately in different entities, and are administered in combination to a prostate cancer patient in therapeutically effective amounts simultaneously, separately, or sequentially; or the compound or the pharmaceutically acceptable salt, the isomer, or the crystal form thereof according to the first or second aspect of the present disclosure, and the novel hormone therapy drug according to the first or second aspect of the present disclosure in the pharmaceutical combination or the pharmaceutical composition or the kit are provided in the form of one inseparable entity, and are administered simultaneously to a prostate cancer patient in a therapeutically effective amount.

In any embodiment of the above fourth, fifth, and sixth aspects of the present disclosure, the prostate cancer is metastatic prostate cancer.

In any embodiment of the above fourth, fifth, and sixth aspects of the present disclosure, the prostate cancer is non-metastatic prostate cancer.

In any embodiment of the above fourth, fifth, and sixth aspects of the present disclosure, the prostate cancer is early-stage prostate cancer.

In any embodiment of the above fourth, fifth, and sixth aspects of the present disclosure, the prostate cancer is advanced prostate cancer.

In any embodiment of the above fourth, fifth, and sixth aspects of the present disclosure, the prostate cancer includes prostate cancer that has been previously treated or that has not received any treatment, wherein the treatment includes standard treatment and/or non-standard treatment.

In any embodiment of the above fourth, fifth, and sixth aspects of the present disclosure, the prostate cancer is prostate cancer that has not previously received standard treatment.

In any embodiment of the above fourth, fifth, and sixth aspects of the present disclosure, the prostate cancer is prostate cancer that has previously failed standard treatment.

In any embodiment of the above fourth, fifth, and sixth aspects of the present disclosure, the prostate cancer is prostate cancer that has previously failed chemotherapy.

In any embodiment of the above fourth, fifth, and sixth aspects of the present disclosure, the prostate cancer is prostate cancer that has previously failed hormone therapy.

In any embodiment of the above fourth, fifth, and sixth aspects of the present disclosure, the prostate cancer is prostate cancer that has previously failed androgen deprivation therapy.

In any embodiment of the above fourth, fifth, and sixth aspects of the present disclosure, the prostate cancer is prostate cancer that has previously failed treatment with an androgen synthesis inhibitor and/or an androgen receptor inhibitor.

In any embodiment of the above fourth, fifth, and sixth aspects of the present disclosure, the prostate cancer is prostate cancer that has previously failed treatment with abiraterone or abiraterone acetate.

In any embodiment of the above fourth, fifth, and sixth aspects of the present disclosure, the prostate cancer is prostate cancer that has previously failed treatment with enzalutamide, apalutamide, darolutamide, and/or bicalutamide.

In any embodiment of the above fourth, fifth, and sixth aspects of the present disclosure, the prostate cancer is prostate cancer that has previously failed treatment with rezvilutamide.

In any embodiment of the above fourth, fifth, and sixth aspects of the present disclosure, the prostate cancer is early-stage and/or non-metastatic prostate cancer, wherein, for example, the early-stage and/or non-metastatic prostate cancer is one that has not previously received standard treatment.

In any embodiment of the above fourth, fifth, and sixth aspects of the present disclosure, the prostate cancer is advanced and/or metastatic prostate cancer, wherein, for example, the advanced and/or metastatic prostate cancer is one that has not previously received standard treatment, or the advanced and/or metastatic prostate cancer is one that has previously failed standard treatment.

In any embodiment of the above fourth, fifth, and sixth aspects of the present disclosure, the prostate cancer is castration-sensitive prostate cancer. In one embodiment, the castration-sensitive prostate cancer is one that has not previously received standard treatment. In one embodiment, the castration-sensitive prostate cancer is one that has previously failed standard treatment. In one embodiment, the castration-sensitive prostate cancer is one that has previously failed chemotherapy. In one embodiment, the castration-sensitive prostate cancer is one that has previously failed hormone therapy. In one embodiment, the castration-sensitive prostate cancer is one that has previously failed androgen deprivation therapy. In one embodiment, the castration-sensitive prostate cancer is one that has previously failed treatment with an androgen synthesis inhibitor and/or an androgen receptor inhibitor. In one embodiment, the castration-sensitive prostate cancer is one that has previously failed treatment with abiraterone or abiraterone acetate. In one embodiment, the castration-sensitive prostate cancer is one that has previously failed treatment with enzalutamide, apalutamide, darolutamide, and/or bicalutamide. In one embodiment, the castration-sensitive prostate cancer is one that has previously failed treatment with rezvilutamide.

In any embodiment of the above fourth, fifth, and sixth aspects of the present disclosure, the prostate cancer is castration-resistant prostate cancer. In one embodiment, the castration-resistant prostate cancer is one that has previously failed standard treatment. In one embodiment, the castration-resistant prostate cancer is one that has previously failed chemotherapy. In one embodiment, the castration-resistant prostate cancer is one that has previously failed hormone therapy. In one embodiment, the castration-resistant prostate cancer is one that has previously failed androgen deprivation therapy. In one embodiment, the castration-resistant prostate cancer is one that has previously failed treatment with an androgen synthesis inhibitor and/or an androgen receptor inhibitor. In one embodiment, the castration-resistant prostate cancer is one that has previously failed treatment with abiraterone or abiraterone acetate. In one embodiment, the castration-resistant prostate cancer is one that has previously failed treatment with enzalutamide, apalutamide, darolutamide, and/or bicalutamide. In one embodiment, the castration-resistant prostate cancer is one that has previously failed treatment with rezvilutamide.

In any embodiment of the above fourth, fifth, and sixth aspects of the present disclosure, the prostate cancer is metastatic castration-sensitive prostate cancer, wherein, for example, the metastatic castration-sensitive prostate cancer is one that has not previously received standard treatment or that has previously failed standard treatment.

In any embodiment of the above fourth, fifth, and sixth aspects of the present disclosure, the prostate cancer is localized prostate cancer, wherein, for example, the localized prostate cancer is one that has not previously received standard treatment or that has previously failed standard treatment.

In any embodiment of the above fourth, fifth, and sixth aspects of the present disclosure, the prostate cancer is metastatic castration-resistant prostate cancer. In one embodiment, the metastatic castration-resistant prostate cancer is one that has previously failed standard treatment. In one embodiment, the metastatic castration-resistant prostate cancer is one that has previously failed chemotherapy. In one embodiment, the metastatic castration-resistant prostate cancer is one that has previously failed hormone therapy. In one embodiment, the metastatic castration-resistant prostate cancer is one that has previously failed androgen deprivation therapy. In one embodiment, the metastatic castration-resistant prostate cancer is one that has previously failed treatment with an androgen synthesis inhibitor and/or an androgen receptor inhibitor. In one embodiment, the metastatic castration-resistant prostate cancer is one that has previously failed treatment with abiraterone or abiraterone acetate. In one embodiment, the metastatic castration-resistant prostate cancer is one that has previously failed treatment with enzalutamide, apalutamide, darolutamide, and/or bicalutamide. In one embodiment, the metastatic castration-resistant prostate cancer is one that has previously failed treatment with rezvilutamide. In one embodiment, the metastatic castration-resistant prostate cancer is histopathologically or cytologically confirmed prostate cancer that has previously failed initial continuous androgen deprivation therapy and has been confirmed by imaging to have a distinct bone or soft tissue metastatic lesion. In one embodiment, the metastatic castration-resistant prostate cancer is histopathologically or cytologically confirmed metastatic castration-resistant prostate cancer for which no standard treatment options are available.

In any embodiment of the above fourth, fifth, and sixth aspects of the present disclosure, the prostate cancer is non-metastatic castration-resistant prostate cancer. In one embodiment, the non-metastatic castration-resistant prostate cancer is one that has previously failed standard treatment. In one embodiment, the non-metastatic castration-resistant prostate cancer is one that has previously failed chemotherapy. In one embodiment, the non-metastatic castration-resistant prostate cancer is one that has previously failed hormone therapy. In one embodiment, the non-metastatic castration-resistant prostate cancer is one that has previously failed androgen deprivation therapy. In one embodiment, the non-metastatic castration-resistant prostate cancer is one that has previously failed treatment with an androgen synthesis inhibitor and/or an androgen receptor inhibitor. In one embodiment, the non-metastatic castration-resistant prostate cancer is one that has previously failed treatment with abiraterone or abiraterone acetate. In one embodiment, the non-metastatic castration-resistant prostate cancer is one that has previously failed treatment with enzalutamide, apalutamide, darolutamide, and/or bicalutamide. In one embodiment, the non-metastatic castration-resistant prostate cancer is one that has previously failed treatment with rezvilutamide.

In any embodiment of the above fourth, fifth, and sixth aspects of the present disclosure, the prostate cancer is metastatic high-risk castration-sensitive prostate cancer.

In any embodiment of the above fourth, fifth, and sixth aspects of the present disclosure, the prostate cancer is non-metastatic castration-sensitive prostate cancer with biochemical recurrence and a high risk of metastasis.

In any embodiment of the above fourth, fifth, and sixth aspects of the present disclosure, the prostate cancer is high-risk metastatic hormone therapy-sensitive prostate cancer. In one embodiment, the high-risk metastatic hormone therapy-sensitive prostate cancer is one that has not previously received standard treatment. In one embodiment, the high-risk metastatic hormone therapy-sensitive prostate cancer is one that has not previously received hormone therapy or has received hormone therapy for no longer than 3 months. In one embodiment, the high-risk metastatic hormone therapy-sensitive prostate cancer is one that has previously failed chemotherapy.

In any embodiment of the above fourth, fifth, and sixth aspects of the present disclosure, the chemotherapy includes docetaxel chemotherapy, docetaxel in combination with a corticosteroid, cabazitaxel chemotherapy, and platinum-based monotherapy or combination chemotherapy.

In any embodiment of the above fourth, fifth, and sixth aspects of the present disclosure, the treatment with abiraterone or abiraterone acetate includes treatment with abiraterone or abiraterone acetate, and treatment with abiraterone or abiraterone acetate in combination with a corticosteroid.

In any embodiment of the above fourth, fifth, and sixth aspects of the present disclosure, the hormone therapy includes androgen deprivation therapy and novel hormone therapy. For example, the androgen deprivation therapy includes surgical castration therapy (such as orchiectomy) and medical castration therapy (including but not limited to the administration of goserelin, leuprorelin, triptorelin, degarelix, etc.).

In any embodiment of the above fourth, fifth, and sixth aspects of the present disclosure, a single-administration dose (calculated based on the compound) of the compound or the pharmaceutically acceptable salt, the isomer, or the crystal form thereof according to the first or second aspect of the present disclosure, i.e., the component (a), is 3-20 mg, such as 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The frequency of the administration is once daily, twice daily, once every two days, or once every three days.

In any embodiment of the above fourth, fifth, and sixth aspects of the present disclosure, the novel hormone therapy drug according to the first or second aspect of the present disclosure, i.e., the component (b), is administered at a dose of 10-2000 mg daily.

In any embodiment of the above fourth, fifth, and sixth aspects of the present disclosure, the component (b) is abiraterone or abiraterone acetate, which is administered at a dose (calculated based on abiraterone) of 500-1500 mg daily, such as 500 mg, 600 mg, 700 mg, 800 mg, 900 mg, 1000 mg, 1100 mg, 1200 mg, 1300 mg, 1400 mg, or 1500 mg daily.

In any embodiment of the above fourth, fifth, and sixth aspects of the present disclosure, the component (b) is abiraterone or abiraterone acetate, which is administered at a dose (calculated based on abiraterone) of 900-1100 mg daily.

In any embodiment of the above fourth, fifth, and sixth aspects of the present disclosure, the component (b) is enzalutamide, which is administered at a dose of 140-180 mg daily, such as 140 mg, 150 mg, 160 mg, 170 mg, or 180 mg daily.

In any embodiment of the above fourth, fifth, and sixth aspects of the present disclosure, the component (b) is enzalutamide, which is administered at a dose of 150-170 mg daily.

In some embodiments of the above fourth, fifth, and sixth aspects of the present disclosure, the component (a) and the component (b) may be provided in the form of one inseparable entity, or provided separately in different entities. When the component (a) and the component (b) are provided in the form of one inseparable entity, each entity may comprise 3-20 mg (e.g., 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg) of the component (a) and 10-2000 mg of the component (b) (e.g., 500-1500 mg or 900-1100 mg, such as 500 mg, 600 mg, 700 mg, 800 mg, 900 mg, 1000 mg, 1100 mg, 1200 mg, 1300 mg, 1400 mg, or 1500 mg of abiraterone or abiraterone acetate (calculated based on abiraterone); or 140-180 mg or 150-170 mg, such as 140 mg, 150 mg, 160 mg, 170 mg, or 180 mg of enzalutamide), or each entity may comprise 1/n of or p times the above amount of the component (a) and 1/m of or q times the above amount of the component (b), wherein n and m are each independently an integer of 1-6, and p and q are each independently an integer of 2-6. When the component (a) and the component (b) are provided separately in different entities, the entity comprising the component (a) may comprise 3-20 mg (e.g., 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg) of the component (a) or 1/n of or p times the above amount of the component (a); and the entity comprising the component (b) may comprise 10-2000 mg of the component (b) (e.g., 500-1500 mg or 900-1100 mg, such as 500 mg, 600 mg, 700 mg, 800 mg, 900 mg, 1000 mg, 1100 mg, 1200 mg, 1300 mg, 1400 mg, or 1500 mg of abiraterone or abiraterone acetate (calculated based on abiraterone); or 140-180 mg or 150-170 mg, such as 140 mg, 150 mg, 160 mg, 170 mg, or 180 mg of enzalutamide) or 1/m of or q times the above amount of the component (b), wherein n and m are each independently an integer of 1-6, and p and q are each independently an integer of 2-6.

In any embodiment of the above fourth, fifth, and sixth aspects of the present disclosure, the pharmaceutical combination or the pharmaceutical composition or the kit may also be administered in combination with a corticosteroid to a prostate cancer patient. In one embodiment, the corticosteroid is prednisone or dexamethasone. For example, the prednisone is administered at a dose of 5-20 mg daily, such as 5 mg daily or 10 mg daily; the dexamethasone is administered at a dose of 0.2-2 mg daily, such as 0.5 mg daily.

In any embodiment of the above fourth, fifth, and sixth aspects of the present disclosure, the compound or the pharmaceutically acceptable salt, the isomer, or the crystal form thereof according to the first or second aspect of the present disclosure, i.e., the component (a), and/or the novel hormone therapy drug according to the first or second aspect of the present disclosure, i.e., the component (b), may also be formulated into any pharmaceutically acceptable pharmaceutical formulation together with one or more pharmaceutically acceptable carriers. The component (a) and the component (b) may be provided in the form of one inseparable entity, or provided separately in different entities. The pharmaceutical formulation may comprise one or more pharmaceutically acceptable carriers, and may be administered to a patient or subject in need of such treatment via a route such as oral, parenteral, rectal, or pulmonary administration. The term "pharmaceutically acceptable carrier" used herein refers to a carrier (or excipient) that does not cause significant irritation to an organism and does not eliminate the biological activity and properties of the administered compound. Any commonly used pharmaceutically acceptable carrier may be used, the choice of which depends on factors such as the particular mode of administration, the effect of the carrier on solubility and stability, and the nature of the dosage form, and is within the ordinary skill of those skilled in the art. Examples of pharmaceutically acceptable carriers include, but are not limited to: fillers (e.g., lactose, sucrose, mannitol or sorbitol, cellulose derivatives, and/or calcium phosphate), binders (e.g., corn, wheat or potato starch, methyl cellulose, hydroxypropyl methyl cellulose, sodium carboxymethyl cellulose, and/or polyvinylpyrrolidone), disintegrants (e.g., starch, carboxymethyl starch, cross-linked polyvinylpyrrolidone, alginic acid or salts thereof), lubricants (e.g., silicic acid, talc, stearic acid or salts thereof, and/or polyethylene glycol or derivatives thereof), diluents, wetting agents, coloring agents (dyes or pigments such as titanium dioxide, lacquer solutions), perfuming agents, absorption enhancers, surfactants, adsorption carriers, etc., such as gelatin, gum tragacanth, gum arabic, carbopol gel, glycerin, sorbitol, liquid paraffin, liquid polyethylene glycol, fatty oils such as sesame oil, or synthetic fatty acid esters such as ethyl oleate or triglycerides, or liposomes.

In any embodiment of the above second aspect of the present disclosure, the component (a) and the component (b) in the pharmaceutical composition may be provided in the form of one inseparable entity, or provided separately in different entities. In any embodiment of the above third aspect of the present disclosure, the a) and the b) in the kit may also be provided in the form of one inseparable entity, or provided separately in different entities. Each of the entities described above may comprise about 0.1-99.5 wt% of the active ingredient, for example, about 0.1-95 wt%, about 0.1-50 wt%, or about 1-40 wt%, such as about 1 wt%, about 1.5 wt%, about 2 wt%, about 5 wt%, about 10 wt%, about 15 wt%, about 20 wt%, about 25 wt%, about 30 wt%, about 40 wt%, or about 50 wt% of the active ingredient, with the remainder being a pharmaceutically acceptable carrier. The entity may comprise one, two, three, or more pharmaceutically acceptable carriers. For oral administration, the entity may be formulated into a conventional solid formulation, such as a tablet, a capsule, a pill, or a granule, or may also be formulated into an oral liquid formulation, such as an oral solution, an oral suspension, or a syrup. In the preparation of an oral formulation, an appropriate filler, binder, disintegrant, lubricant, etc., may be added. For parenteral administration, the entity may be formulated into an injection, including a solution injection, a sterile powder for injection, and a concentrated solution for injection. The injection may be produced by a conventional method existing in the pharmaceutical field, and during the formulation process, additives may not be added, or an appropriate additive (e.g., a physiologically compatible buffer such as Hanks' solution, Ringer's solution, or normal saline buffer) may be added based on the properties of the drug. For rectal administration, the entity may be formulated into a suppository or the like (e.g., containing conventional suppository base materials such as cocoa butter or other glycerides). For pulmonary administration, the entity may be formulated into an inhalant, a spray, or the like (e.g., delivered in the form of an aerosol spray from a pressurized pack or a nebulizer, with the aid of a suitable propellant, such as dichlorodifluoromethane, trichlorofluoromethane, dichlorotetrafluoroethane, carbon dioxide, or other suitable gas).

### Beneficial Effects of the Present Disclosure

Through research, it has been found that the compound of general formula (I) or the pharmaceutically acceptable salt, the isomer, or the crystal form thereof of the present disclosure, in combination with the novel hormone therapy drug, exhibits a synergistic effect in the treatment of prostate cancer. Further, compound 29 of the present disclosure, in combination with a specific novel hormone therapy drug, exhibits a synergistic effect in the treatment of prostate cancer. Furthermore, compound 29 of the present disclosure, in combination with enzalutamide or abiraterone acetate, exhibits a significant synergistic effect in the treatment of prostate cancer, especially metastatic castration-resistant prostate cancer (mCRPC) with distant metastasis.

### Detailed Description of the Present Disclosure

The "halogen" described herein refers to fluorine, chlorine, bromine, iodine, and the like, preferably fluorine and chlorine.

The "halogenated" described herein means that any hydrogen atom in a substituent may be replaced by one or more identical or different halogen atoms. The "halogen" is as defined above.

The "cyano" described herein refers to the -CN group.

The "amino" described herein refers to the -NH₂ group.

The "C₁₋₄ alkyl" described herein refers to linear or branched alkyl derived by removing one hydrogen atom from an alkane moiety containing 1-4 carbon atoms, such as methyl, ethyl, *n*-propyl, isopropyl, *n*-butyl, isobutyl, *sec*-butyl, or *tert-*butyl*.* The "C₁₋₃ alkyl" refers to the above alkyl containing 1-3 carbon atoms.

The "C₃₋₆ cycloalkyl" described herein refers to a monocyclic cycloalkyl group, a bicyclic cycloalkyl system or a polycyclic cycloalkyl system containing 3-6 carbon atoms. Such groups are saturated but not aromatic. Unless otherwise specified, monocyclic and polycyclic ring structures that can be formed are included. Examples of the C₃₋₆ cycloalkyl include, but are not limited to: cyclopropyl, cyclobutyl, cyclopentyl, and cyclohexyl.

The "5-11 membered heterocyclyl" described herein refers to a non-aromatic cyclic group having 5-11 ring carbon atoms, wherein at least one ring carbon atom is replaced by one or more heteroatoms (preferably 1-3 heteroatoms) selected from O, S, and N, and the ring-forming atoms including carbon atoms, nitrogen atoms, and sulfur atoms may be oxidized.

The "heterocyclyl" refers to a monocyclic heterocyclyl group, a bicyclic heterocyclyl system, or a polycyclic heterocyclyl system, including saturated and partially saturated heterocyclyl, but excluding aromatic rings. Unless otherwise specified, the "5-11 membered heterocyclyl" described herein includes monocyclic and polycyclic ring structures that can be formed.

The monocyclic heterocyclyl may be 5-7 membered heterocyclyl, 5-6 membered heterocyclyl, 5-6 membered oxygen-containing heterocyclyl, 5-6 membered nitrogen-containing heterocyclyl, 5-6 membered saturated heterocyclyl, etc. Examples of the 5-6 membered monocyclic heterocyclyl described herein include, but are not limited to, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H-*imidazolyl, 4,5-dihydro-1*H*-imidazolyl, 4,5-dihydro-1*H*-pyrazolyl, 4,5-dihydro-3*H-*pyrazolyl, 4,5-dihydrothiazolyl, 2,5-dihydrothiazolyl, 2*H*-pyranyl, 4H-pyranyl, *2H-*thiopyranyl, 4*H*-thiopyranyl, 2,3,4,5-tetrahydropyridinyl, 1,2-isoxazinyl, 1,4-isoxazinyl, 6*H-*1,3-oxazinyl, etc.

The polycyclic heterocyclyl includes ortho-fused heterocyclyl, spiro-heterocyclyl, and bridged heterocyclyl, which may be saturated, partially saturated, or unsaturated, but not aromatic. Unless otherwise specified, the 7-11 membered polycyclic heterocyclyl described herein includes ortho-fused, spiro-, and bridged structures that can be formed.

The ortho-fused heterocyclyl may be 7-11 membered ortho-fused cyclyl, preferably 7-11 membered saturated ortho-fused cyclyl, and examples thereof include, but are not limited to: 3,6-diazabicyclo[3.2.0]heptane, 3,8-diazabicyclo[4.2.0]octanyl, 3,7-diazabicyclo[4.2.0]octanyl, octahydropyrrolo[3,4-*c*]pyrrole, octahydropyrrolo[3,4-*b*]pyrrole, octahydropyrrolo[3,4-*b*][1,4]oxazinyl, octahydro-1*H*-pyrrolo[3,4-c]pyridine, 2,3-dihydrobenzofuran-2-yl, 2,3-dihydrobenzofuran-3-yl, indolin-1-yl, indolin-2-yl, indolin 3-yl, 2,3-dihydrobenzothien-2-yl, octahydro-1*H*-indolyl, and octahydrobenzofuranyl.

The spiro-heterocyclyl may be 7-11 membered spiro-heterocyclyl, preferably 7-11 membered saturated spiro-heterocyclyl, and examples thereof include, but are not limited to:

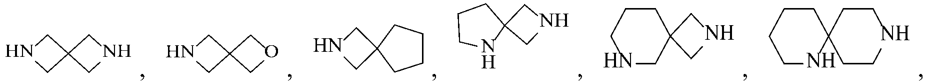

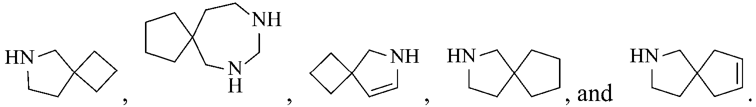

The bridged heterocyclyl may be 7-11 membered bridged heterocyclyl, preferably 7-11 membered saturated bridged heterocyclyl, and examples thereof include, but are not limited to:

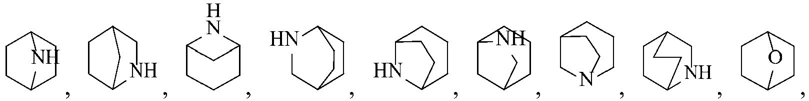

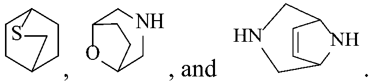

The "pharmaceutically acceptable salt" described herein refers to pharmaceutically acceptable acid and base addition salts and solvates. Such pharmaceutically acceptable salts include salts of acids such as: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, and alkanoic acids (such as acetic acid and HOOC-(CH₂)ₙ-COOH (where n = an integer of 0-4)). Such pharmaceutically acceptable salts further include salts of bases such as: sodium, potassium, calcium, and ammonium. Those skilled in the art know a variety of non-toxic pharmaceutically acceptable addition salts.

Each numerical range described herein includes both endpoints of the range, all integers within the range, and subranges formed by these integers. For example, "5-11 membered" includes 5, 6, 7, 8, 9, 10, or 11 membered, "5-6 membered" includes 5 or 6 membered, "7-11 membered" includes 7, 8, 9, 10, or 11 membered, and so on.

The "one or more" described herein with respect to a substituent refers to the number of substituents with which all positions can be chemically substituted in the substituted group, preferably 1-6, more preferably 1-5, more preferably 1-3, and even more preferably 1-2.

The "crystal form" described herein may be prepared from the compound of general formula (I) by conventional methods for preparing crystal forms used in the art.

The "isomer" of the compound of general formula (I) described herein includes stereoisomers and tautomers.

The term "stereoisomer" refers to isomers resulting from different spatial arrangements of atoms in a molecule. When asymmetric carbon atoms are present in a compound, enantiomers may be formed; when a carbon-carbon double bond or a cyclic structure is present in a compound, cis-trans isomers may be formed.

The term "tautomer" means that different functional group isomers are in dynamic equilibrium and can be rapidly converted into each other, representing a special type of functional group isomerism. For example, when a ketone or oxime is present, tautomers may be formed; representative examples include: keto-enol tautomers, phenol-keto tautomers, nitroso-oxime tautomers, imine-enamine tautomers, etc.

All enantiomers, diastereomers, racemates, cis-trans isomers, tautomers, geometric isomers, and epimers of the compounds, as well as mixtures thereof, are included within the scope of the present disclosure.

For the preparation of the compound of general formula (I) described herein, reference may be made to the detailed description section of WO2018108079A1.

The "treat", "treating", or "treatment" described herein refers to administering an effective amount of a drug to a patient to slow down or cure an undesired physiological change or condition such as a hyperproliferative condition (e.g., the growth, formation, or spread of a cancer) in the patient, thereby achieving a favorable or desired clinical outcome, including but not limited to: alleviation of symptoms, reduction in the disease severity, stabilization (i.e., not worsening) of the disease state, delay or slowing down of the disease progression, amelioration or mitigation of the disease state, and regression (whether partial or complete), whether detectable or undetectable.

The "treat", "treating", or "treatment" described herein may include neoadjuvant treatment and adjuvant treatment.

The "neoadjuvant treatment" refers to systemic treatment for a patient with an initial tumor prior to planned surgery or local treatment with surgery plus radiotherapy; depending on the tumor type, the neoadjuvant treatment may be performed by means of chemotherapy, hormone therapy, targeted therapy, immunotherapy, or radiotherapy. The purpose of the neoadjuvant treatment is to provide immediate systemic treatment, thereby potentially eradicating micrometastases that would otherwise proliferate under the standard sequence of surgery followed by systemic therapy. The neoadjuvant treatment may also help reduce tumor size, thereby allowing complete resection of an initially unresectable tumor or preservation of a portion of the organ and its function. In addition, the neoadjuvant treatment allows for *in vivo* evaluation of drug efficacy, which may guide the selection of subsequent treatments.

The "adjuvant treatment" refers to a therapy given after radical surgery (where no evidence of residual disease is detected) to eliminate any remaining cancer cells in the body, thereby reducing the likelihood of tumor recurrence or dissemination to other sites. The means of the adjuvant treatment are roughly the same as those of the neoadjuvant treatment. The objective of the adjuvant treatment is to prevent cancer recurrence and thus reduce the chance of cancer-related death. In this context, the adjuvant treatment explicitly excludes the neoadjuvant treatment.

The "used in combination", "combined use", "administered in combination", or "combination therapy" described herein refers to the administration of at least one dose of the compound of general formula (I) or the pharmaceutically acceptable salt, the isomer, or the crystal form thereof described above and the novel hormone therapy drug described above with a certain period of time, wherein the components may be formulated together into a compound formulation or formulated separately into individual formulations for simultaneous, separate, or sequential administration to a patient; or on the basis of the combination described above, a corticosteroid may be administered to the patient (simultaneously, separately, or sequentially).

The "failed", "failed treatment", or "failed chemotherapy" described herein refers to the occurrence of progressive disease (progressive disease (PD) assessed according to the RECIST 1.1 response evaluation criteria for tumor tissue assessment or progressive disease (PD) assessed according to the PCWG3 criteria for bone lesion evaluation in prostate cancer) during the treatment or after the last treatment, or intolerance due to toxic side effects during the treatment.

The "drug resistance" described herein refers to a decrease in the sensitivity of a pathogen, a tumor cell, or an individual to a drug, resulting in the drug failing to exert the intended therapeutic effect, which aligns with the situation of the "failed treatment" described herein.

The "intolerance due to toxic side effects" described herein refers to the inability to continue the treatment due to adverse reactions caused by the drug.

The "standard treatment" described herein includes androgen deprivation therapy (surgical castration and medical castration therapies including but not limited to goserelin, leuprorelin, triptorelin, degarelix, etc.), chemotherapy, systemic radiotherapy, and/or novel hormone therapy. Of course, the "standard treatment" is not limited to those mentioned above. As the standard treatment regimens are updated, the "standard treatment" described herein will encompass all such updates.

The "non-standard treatment" described herein refers to treatment performed by means other than standard treatment.

The "chemotherapy" described herein includes, but is not limited to, docetaxel, docetaxel in combination with a corticosteroid (e.g., prednisone), cabazitaxel, platinum-based monotherapy or combination chemotherapy regimens, etc.

The "treatment with abiraterone acetate" described herein includes, but is not limited to, abiraterone acetate, abiraterone acetate in combination with a corticosteroid (e.g., prednisone), etc.

The "hormone therapy" described herein is a method for treating prostate cancer by lowering androgen levels and inhibiting or reducing androgen receptor activity. It includes both traditional androgen deprivation therapy (ADT) and novel hormone therapy (NHT). Among them, ADT includes surgical castration therapy (such as orchiectomy) and medical castration therapy (including but not limited to the administration of goserelin, leuprorelin, triptorelin, degarelix, etc.).

The "therapeutically effective amount" described herein refers to an amount of the compound or the pharmaceutically acceptable salt, the isomer, or the crystal form thereof and the novel hormone therapy drug described above that, when administered to a patient, is at least capable of alleviating symptoms of the patient's condition. An actual amount comprising the "therapeutically effective amount" will vary depending on multiple factors, including but not limited to the particular condition being treated, the severity of the condition, the physique and health status of the patient, and the route of administration. For example, a single bolus dose may be administered, several separate doses may be administered over time, or the dose may be proportionally reduced or increased as necessitated by the exigencies of the treatment situation. It should be noted that the dose values may vary with the type and severity of the condition to be alleviated, and may include single or multiple doses. For any particular individual, the specific dosing regimen should be adjusted over time according to the individual's needs and the professional judgment of the person administering the composition or supervising the administration of the composition.

The "unit formulation" described herein refers to a physically discrete unit suitable for use as a unit dose for human subjects and other mammals, wherein each unit contains a predetermined amount of active substance calculated to produce the desired therapeutic effect, as well as a suitable pharmaceutical excipient. Typical unit formulations include pre-filled, pre-measured ampoules or syringes for liquid compositions, or, in the case of solid compositions, pills, tablets, capsules, lozenges, etc. In such a composition, the active substance is usually a component with a small amount (e.g., about 0.1 wt% to about 50 wt%, or preferably about 1 wt% to about 40 wt%), with the remainder consisting of various vehicles or carriers and processing aids that facilitate the formation of the desired dosage form. Unit dose formulations are preferably about 3 mg, 4 mg, 5 mg, 6 mg, 7 mg, 8 mg, 9 mg, 10 mg, 25 mg, 50 mg, 100 mg, 250 mg, 500 mg, 1000 mg, or the like per unit. In a specific embodiment, unit dosage forms are packaged in a multipack for sequential use, such as a blister pack containing at least 6, 9, or 12 unit dosage form tablets.

The "subject" or "individual" in the prevention and/or treatment of prostate cancer refers to any animal classified as a mammal, including humans, domesticated and farm animals, and zoo, sports, or pet animals, such as dogs, horses, cats, and cattle. Preferably, the mammal is a human. The subject or individual may be a patient.

The "synergistic effect" described herein refers to an effect produced by two active ingredients (e.g., the compound of general formula (I) and the novel hormone therapy drug), the positive effects of which include: an effect greater than the additive effect, additional beneficial effects (e.g., additional therapeutic effects not previously observed with either ingredient alone), reduced side effects, achievement of a combined therapeutic effect produced by a non-effective dose of one or both of the first and second active ingredients, and a stronger synergistic interaction between the two active ingredients. The positive effects of the synergistic effect are not limited to those mentioned above, and may also include other positive effects.

In the art, "abiraterone acetate" is sometimes referred to as "abiraterone".

### DETAILED DESCRIPTION

In order to make the objective, technical solutions, and advantages of the present disclosure more apparent, the present disclosure is further illustrated in detail below. It is apparent that the described examples are only a part of the examples of the present disclosure, but not all of them. All other examples obtained by those of ordinary skill in the art based on the examples in the present disclosure without making inventive efforts shall fall within the protection scope of the present disclosure.

The abbreviations and English expressions used in the present disclosure have the following meanings:

| Abbreviation/English | Meaning |
|---|---|
| DMSO | Dimethyl sulfoxide |
| MC | Methyl cellulose |
| Qd | Once-daily administration |
| mCRPC | Metastatic castration-resistant prostate cancer |
| PEG300 | Polyethylene glycol 300 |
| PR | Partial response |
| CR | Complete response |
| SD | Stable disease |
| PD | Progressive disease |

### Examples

DMSO, MC, and PEG300 used in the examples were all obtained from commercially available sources.

Experimental Example 1: *In Vivo* Pharmacodynamic Study of Compound of the Present Disclosure in Combination with Enzalutamide on Subcutaneous Xenograft Tumor Model of Human Prostate Cancer Cell Line

Test substances: Compound 29 of the present disclosure, whose structure is shown above, was prepared with reference to the detailed description section of WO2018108079A1. Enzalutamide was obtained from a commercially available source.

Materials such as information on animal and cell line sources:
The cell line used for the human prostate cancer model in this experiment was 22Rv1, purchased from ATCC.

Male BALB/c nude mice, aged 6-8 weeks, were obtained from SPF (Beijing) Biotechnology Co., Ltd.

### Test method:

### 1. Construction and grouping of tumor-bearing mice

Fourteen days before tumor cell inoculation, all mice were castrated via scrotal excision of the testes. 22Rv1 tumor cells were subcutaneously injected into the right flank of each mouse along with 0.1 mL of a 1:1 mixture of serum-free RPMI-1640 medium and Matrigel (supplier: Coming) to cultivate tumors. Before drug administration, the animals were weighed, and the tumor volumes were measured. Grouping was then performed using a block design according to the tumor volumes.

### 2. Dosing regimen

Administration was carried out according to the table below.

| Group | Dose (mg/kg) | Number of animals | Route of administration | Frequency of administration | Administration period |
|---|---|---|---|---|---|
| Vehicle control | 0 | 5 | Oral gavage | Qd * 7 days/week | 28 days |
| Enzalutamide | 30 | 5 | Oral gavage | Qd * 7 days/week | 28 days |
| Compound 29 | 15 | 5 | Oral gavage | Qd * 7 days/week | 28 days |
| Compound 29 + enzalutamide | 15 +30 | 5 | Oral gavage | Qd * 7 days/week | 28 days |
| | | | Oral gavage | Qd * 7 days/week | |

| | | | | | |
|---|---|---|---|---|---|
| Note: Vehicle control: DMSO/PEG300/Tween 80/normal saline (%): 5/40/5/50; formula of compound 29: 0.5% MC; formula of enzalutamide: DMSO/PEG300/Tween 80/normal saline (%): 5/40/5/50. | | | | | |

### 3. Experimental observation indicators

The animals were monitored daily for health status and mortality, body weight and tumor volume were measured twice a week, and samples were collected after the last administration. Response evaluation was performed based on the clinical response evaluation indicators for solid tumors (RECIST1.1). After two consecutive measurements of tumor volume, individual mice with a tumor volume reduction greater than or equal to 30% of the day-0 measurement were considered to have achieved partial response (PR). Individual mice with no palpable tumor (0.00 mm³ for two consecutive measurements) were classified as complete response (CR); individuals with a tumor volume increase less than 20% of the day-0 volume or a tumor volume reduction less than 30% of the day-0 volume were classified as stable disease (SD); individuals with a tumor volume increase greater than 20% of the day-0 measurement were classified as progressive disease (PD). The results are shown in Table 2:

**Table 2. Effect of compound 29 of the present disclosure in combination with enzalutamide on tumor growth in the human prostate cancer 22Rv1 CDX subcutaneous xenograft tumor model**

| Group | Number of animals | Response evaluation (number of animals/number of animals per group) | |
|---|---|---|---|
| | | CR | PR |
| Vehicle control | 5 | 0/5 | 0/5 |
| Enzalutamide | 5 | 0/5 | 0/5 |
| Compound 29 | 5 | 0/5 | 0/5 |
| Compound 29 + enzalutamide | 5 | 1/5 | 1/5 |

As can be seen from the experimental results in Table 2, this cell line was insensitive to enzalutamide treatment, while the combined use of compound 29 and enzalutamide exhibited a significant inhibitory effect on the human prostate cancer 22Rvl castration subcutaneous xenograft tumor model, indicating that the compound of the present disclosure, in combination with enzalutamide, has promising clinical application potential for the treatment of prostate cancer, including enzalutamide treatment-insensitive prostate cancer.

Experimental Example 2: *In Vivo* Pharmacodynamic Study of Compound of the Present Disclosure in Combination with Enzalutamide on Castration Subcutaneous Xenograft Tumor Model of Human Prostate Cancer Cell Line

Test substances: Compound 29 of the present disclosure, whose structure is shown above, was prepared with reference to the detailed description section of WO2018108079A1. Enzalutamide was obtained from a commercially available source.

### Materials such as information on animal and cell line sources:

The cell line used for the human prostate cancer model in this experiment was an enzalutamide-resistant strain (obtained from in-house long-term administration of enzalutamide to a commercially available human prostate cancer cell line in the laboratory).

Male BALB/c nude mice, aged 6-8 weeks, were obtained from Beijing Vital River Laboratory Animal Technology Co., Ltd.

### Test method:

### 1. Construction and grouping of tumor-bearing mice

Fourteen days before tumor cell inoculation, all mice were castrated via scrotal excision of the testes. Tumor cells of the enzalutamide-resistant strain were subcutaneously injected into the right flank of each mouse along with 0.1 ml of a 1:1 mixture of serum-free RPMI-1640 medium and Matrigel to cultivate tumors. Before drug administration, the animals were weighed, and the tumor volumes were measured. Grouping was then performed using a block design according to the tumor volumes.

### 2. Dosing regimen

Administration was carried out according to the table below.

| Group | Dose (mg/kg) | Number of animals | Route of administration | Frequency of administration | Administration period |
|---|---|---|---|---|---|
| Vehicle control | 0 | 5 | Oral gavage | Qd * 7 days/week | 28 days |
| Enzalutamide | 30 | 5 | Oral gavage | Qd * 7 days/week | 28 days |
| Compound 29 | 15 | 5 | Oral gavage | Qd * 7 days/week | 28 days |
| Compound 29 + enzalutamide | 15 +30 | 5 | Oral gavage | Qd * 7 days/week | 28 days |
| | | | Oral gavage | Qd * 7 days/week | |

| | | | | | |
|---|---|---|---|---|---|
| Note: Vehicle control: DMSO/PEG300/Tween 80/normal saline (%): 5/40/5/50; formula of compound 29: 0.5% MC; formula of enzalutamide: DMSO/PEG300/Tween 80/normal saline (%): 5/40/5/50. | | | | | |

### 3. Experimental observation indicators

The animals were monitored daily for health status and mortality, body weight and tumor volume (TV) were measured twice a week, and samples were collected after the last administration. At the end of the study, the tumor growth inhibition rate (TGI%) was calculated for each group based on the tumor volume: TGI% = (1 - T/C) × 100%; T/C% = TRTV/CRTV × 100%. TRTV: mean RTV of treatment group; CRTV: mean RTV of control group; RTV = Vf/Vi, where Vi: TV at initial treatment; Vf: TV after treatment. The results are shown in Table 3:

**Table 3. Effect of compound 29 of the present disclosure in combination with enzalutamide on tumor growth in the human prostate cancer enzalutamide-resistant strain CDX subcutaneous xenograft tumor model**

| Group | Number of animals | Response evaluation | |
|---|---|---|---|
| | | Tumor volume (mm³) | TGI% |
| Vehicle control | 5 | 2066.8±328.5 | |
| Enzalutamide | 5 | 1467.8±213.1 | 27.69 |
| Compound 29 | 5 | 775.0±91.6^{#, ##} | 60.85 |
| Compound 29 + enzalutamide | 5 | 343.2±83.7*^{,} **^{,} *** | 83.14 |

| | | | |
|---|---|---|---|
| # When compound 29 was used alone, the tumor volume showed a statistically significant difference compared with the vehicle control group, *P* < 0.01 ## When compound 29 was used alone, the tumor volume showed a statistically significant difference compared with the enzalutamide group, *P* = 0.028 * When compound 29 was used in combination with enzalutamide, the tumor volume showed a statistically significant difference compared with the vehicle control group, *P* < 0.01 ** When compound 29 was used in combination with enzalutamide, the tumor volume showed a statistically significant difference compared with the enzalutamide group, *P* < 0.01 *** When compound 29 was used in combination with enzalutamide, the tumor volume showed a statistically significant difference compared with the compound 29 monotherapy group, *P* = 0.01 | | | |

As can be seen from the experimental results in Table 3, the combined use of compound 29 and enzalutamide exhibited a significant inhibitory effect on the castration subcutaneous xenograft tumor model of the human prostate cancer enzalutamide-resistant strain, with a significant statistical difference (**, *P* < 0.01), indicating that the compound of the present disclosure, in combination with enzalutamide, has promising clinical application potential for the treatment of enzalutamide-resistant prostate cancer.

Experimental Example 3: Clinical Study of Compound of the Present Disclosure in Combination with Abiraterone Acetate or Enzalutamide in Treatment of Prostate Cancer Patients

### Test drugs:

Compound 29 of the present disclosure, whose structure is shown above, was prepared with reference to the detailed description section of WO2018108079A1.

Abiraterone acetate and enzalutamide were obtained from commercially available sources.

Inclusion criteria: patients with histologically or cytologically confirmed prostate cancer (including those with metastatic castration-resistant prostate cancer for whom no standard treatment options are available).

### Dosing regimen:

Treatment group A: Compound 29 was administered orally at a specified dose (e.g., 5 mg, 6 mg, 8 mg, or 10 mg) once daily; abiraterone acetate was administered orally at a specified dose (e.g., 500 mg, 750 mg, or 1000 mg) once daily.

Treatment group B: Compound 29 was administered orally at a specified dose (e.g., 5 mg, 6 mg, 8 mg, or 10 mg) once daily; enzalutamide was administered orally at a specified dose (e.g., 80 mg, 120 mg, or 160 mg) once daily.

Evaluation of expected efficacy: It was expected that compound 29, in combination with abiraterone acetate or enzalutamide, would have a therapeutic effect in the treatment of prostate cancer in clinical practice.

Experimental Example 4: *In Vivo* Pharmacodynamic Study of Compound of the Present Disclosure on Human Castration-Resistant Prostate Cancer Subcutaneous PDX (Patient-Derived Xenograft) Tumor Model
Test substances: Compound 29 of the present disclosure, whose structure is shown above, was prepared with reference to the detailed description section of WO2018108079A1. Abiraterone was obtained from a commercially available source.

Materials such as information on animals and tumor mass source patient: The human prostate cancer tumor sample LD1-0034-361929 was derived from a male patient. Clinical diagnosis: castration-resistant prostate cancer with bladder metastatic lesions; pathological diagnosis: prostate cancer - poorly differentiated adenocarcinoma.

Male Nu/Nu nude mice aged 6-8 weeks were used.

### Test method:

### 1. Construction and grouping of tumor-bearing mice

The tumor masses were inoculated into the right back of the mice. Before drug administration, the animals were weighed, and the tumor volumes were measured. Grouping was then performed using a block design according to the tumor volumes.

### 2. Dosing regimen

Administration was carried out according to the table below.

| Group | Dose (mg/kg) | Number of animals | Route of administration | Frequency of administration | Administration period |
|---|---|---|---|---|---|
| Vehicle control | 0 | 5 | Oral gavage | Qd * 7 days/week | 39 days |
| Abiraterone | 200 | 5 | Oral gavage | Qd * 7 days/week | 39 days |
| Compound 29 | 15 | 5 | Oral gavage | Qd * 7 days/week | 39 days |
| Compound 29 + abiraterone | 15+ 200 | 5 | Oral gavage | Qd * 7 days/week | 39 days |
| | | | Oral gavage | Qd * 7 days/week | |

| | | | | | |
|---|---|---|---|---|---|
| Note: Vehicle control: 0.5% MC; formula of compound 29: 0.5% MC; formula of abiraterone: 10% DMSO + 90% vegetable oil | | | | | |

### 3. Experimental observation indicators

The animals were monitored daily for health status and mortality, body weight and tumor volume were measured twice a week, and samples were collected after the last administration. The efficacy based on tumor volume size was evaluated using TGI%. The relative tumor growth inhibition rate TGI (%) was calculated as: TGI = 1 - T/C (%). T/C % represents the relative tumor proliferation rate, i.e., the percentage of the relative tumor volume of the treatment group compared to the control group at a given time point. T and C represent the relative tumor volumes (RTVs) of the treatment group and the control group at a given time point, respectively. The calculation formula is as follows: T/C % = TRTV/CRTV × 100% (TRTV: mean RTV of treatment group; CRTV: mean RTV of vehicle control group; RTV = Vt/V0, where V0 is the tumor volume of the animal at the time of grouping, and Vt is the tumor volume of the animal after treatment).

### The results are shown in the table below:

**Table: Effect of compound 29 of the present disclosure in combination with abiraterone on tumor growth in the human prostate cancer LD1-0034-361929 subcutaneous xenograft tumor model**

| Group | Number of animals | Tumor volume (mm³) | TGI (%) |
|---|---|---|---|
| Vehicle control | 5 | 1404.87±483.33 | -- |
| Abiraterone | 5 | 1549.43±533.15 | -8.97 |
| Compound 29 | 5 | 622.23±247.3 | 58.07 |
| Compound 29 + abiraterone | 5 | 203.85±66.15 | 85.15 |

As can be seen from the experimental results in the above table, compared with monotherapy (compound 29 alone or abiraterone alone), the combined use of compound 29 and abiraterone exhibited a significant inhibitory effect on the human castration-resistant prostate cancer PDX subcutaneous xenograft tumor model LD1-0034-361929, indicating that the combination of compound 29 of the present disclosure and abiraterone has promising clinical application potential for the treatment of mCRPC.

The above description is only for the purpose of illustrating preferred examples of the present disclosure, and is not intended to limit the scope of the present disclosure. Any modifications, equivalents, improvements, and the like made without departing from the spirit and principle of the present disclosure should be included in the protection scope of the present disclosure.

## Claims

1. A pharmaceutical combination, comprising: (a) a compound of general formula (I) or a pharmaceutically acceptable salt, an isomer, or a crystal form thereof, and (b) a novel hormone therapy drug; 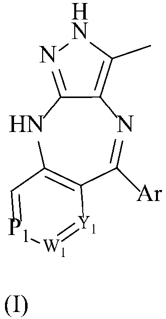
wherein Ar is phenyl optionally substituted with 1-3 R⁶, each R⁶ being independently selected from hydrogen, amino, cyano, halogen, C₁₋₄ alkyl, and trifluoromethyl;
Y₁ is CR³;
P₁ is CR⁴;
W₁ is N;
R³ is hydrogen or C₁₋₄ alkyl;
R⁴ is -(CH₂)ₙ-(5-11) membered heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
further,
Ar is phenyl optionally substituted with 1-3 R⁶, each R⁶ being independently selected from hydrogen and halogen;
Y₁ is CR³, and R³ is hydrogen;
P₁ is CR⁴, and R⁴ is selected from -(CH₂)ₙ-(5-6) membered monocyclic heterocyclyl and -(CH₂)ₙ-(7-11) membered polycyclic heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
W₁ is N.

2. A pharmaceutical composition, comprising: (a) a compound of general formula (I) or a pharmaceutically acceptable salt, an isomer, or a crystal form thereof, and (b) a novel hormone therapy drug; 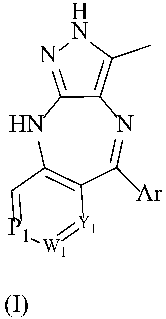
wherein Ar is phenyl optionally substituted with 1-3 R⁶, each R⁶ being independently selected from hydrogen, amino, cyano, halogen, C₁₋₄ alkyl, and trifluoromethyl;
Y₁ is CR³;
P₁ is CR⁴;
W₁ is N;
R³ is hydrogen or C₁₋₄ alkyl;
R⁴ is -(CH₂)ₙ-(5-11) membered heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
further,
Ar is phenyl optionally substituted with 1-3 R⁶, each R⁶ being independently selected from hydrogen and halogen;
Y₁ is CR³, and R³ is hydrogen;
P₁ is CR⁴, and R⁴ is selected from -(CH₂)ₙ-(5-6) membered monocyclic heterocyclyl and -(CH₂)ₙ-(7-11) membered polycyclic heterocyclyl, wherein n = an integer of 0-6, a ring-forming S atom (if any) in the heterocyclyl is optionally oxidized to S(O) or S(O)₂, a ring-forming C atom (if any) in the heterocyclyl is optionally oxidized to C(O), and the heterocyclyl is optionally substituted with one or more substituents independently selected from C₁₋₃ alkyl and C₃₋₆ cycloalkyl;
W₁ is N.

3. The pharmaceutical combination according to claim 1 or the pharmaceutical composition according to claim 2, wherein
R⁴ is 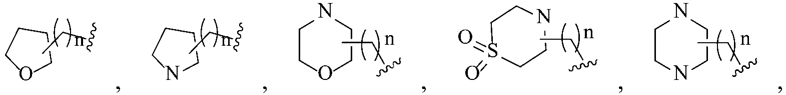 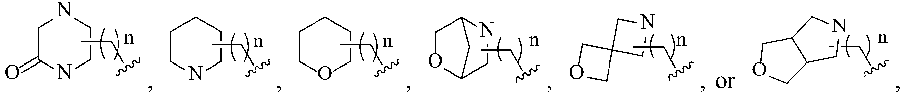 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl); for example, R⁴ is 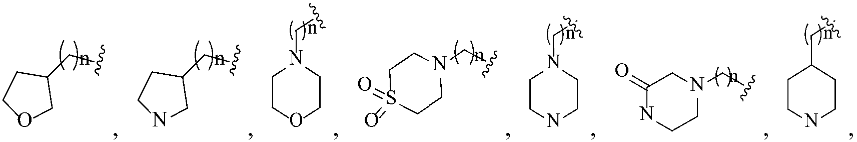 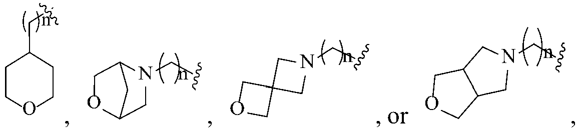 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
further, R⁴ is 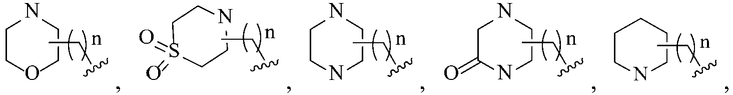 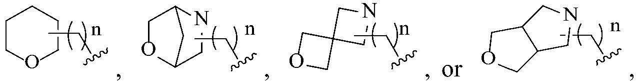 and n = an integer of 0-3, wherein the heterocyclyl in R⁴ is optionally substituted with one or more substituents independently selected from C₁₋₃ alkyl and C₃₋₆ cycloalkyl (for example, 1, 2, or 3 substituents independently selected from C₁₋₂ alkyl and C₃ cycloalkyl);
for example, R⁴ is 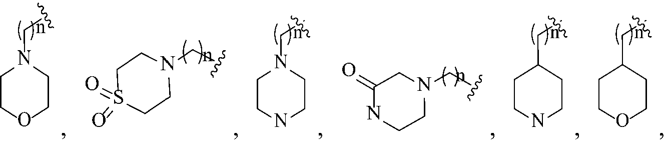 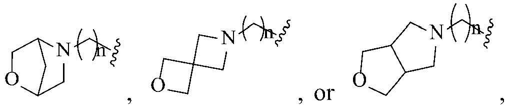 and n = an integer of 0-3, wherein the heterocyclyl in R⁴ is optionally substituted with one or more substituents independently selected from C₁₋₃ alkyl and C₃₋₆ cycloalkyl.

4. The pharmaceutical combination according to claim 1 or the pharmaceutical composition according to claim 2, wherein the component (a) is one or more of the following compounds or pharmaceutically acceptable salts, isomers, or crystal forms thereof: 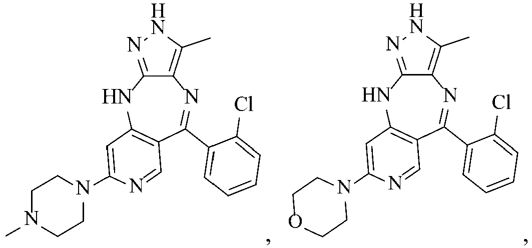 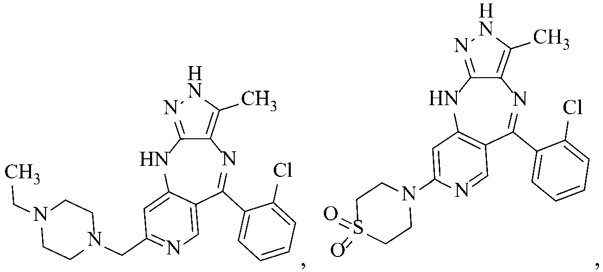 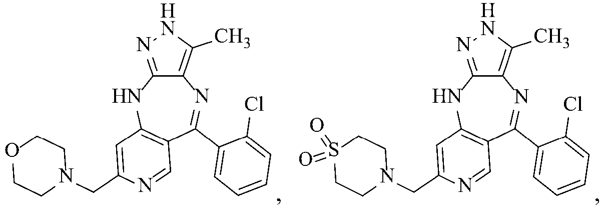 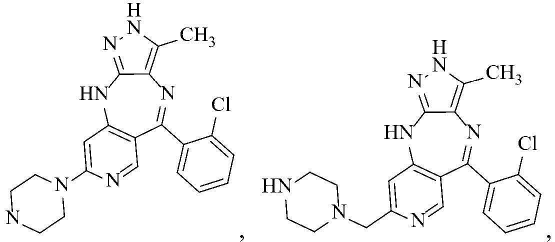 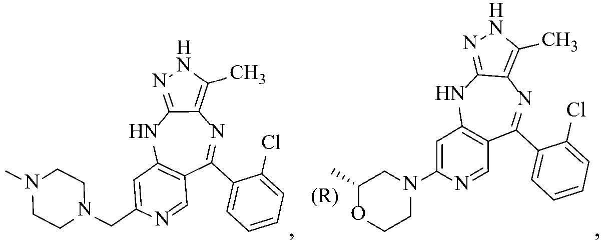 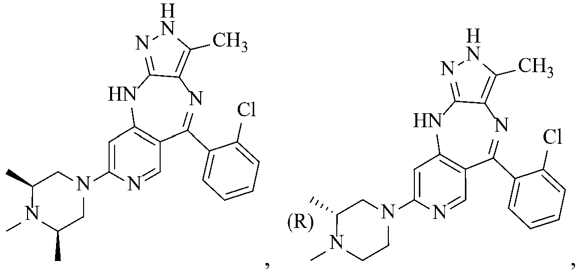 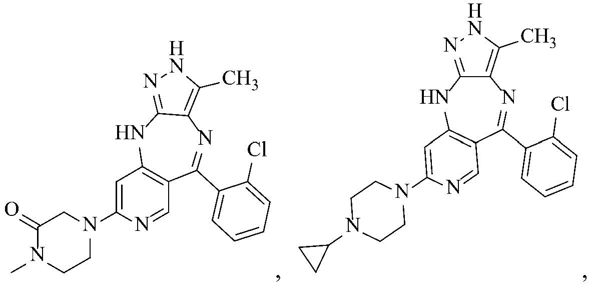 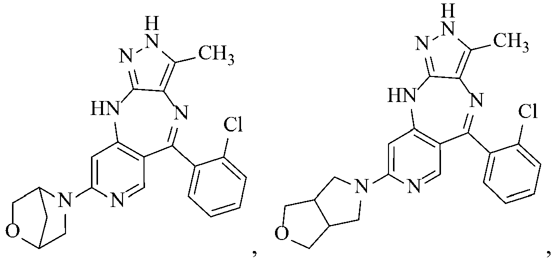 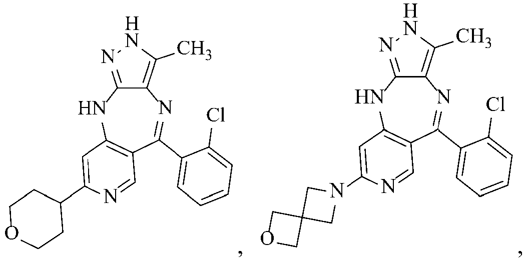 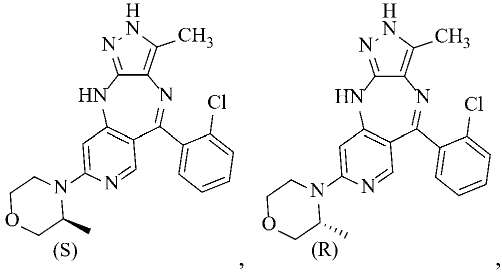 and

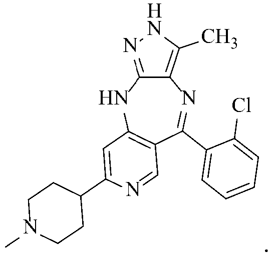

5. The pharmaceutical combination according to any one of claims 1 and 3-4 or the pharmaceutical composition according to any one of claims 2-4, wherein the novel hormone therapy drug is an androgen synthesis inhibitor and/or an androgen receptor inhibitor; preferably, the androgen receptor inhibitor is enzalutamide, apalutamide, darolutamide, bicalutamide, or rezvilutamide, and the androgen synthesis inhibitor is abiraterone or abiraterone acetate.

6. The pharmaceutical combination according to any one of claims 1 and 3-4 or the pharmaceutical composition according to any one of claims 2-4, wherein the (a) is 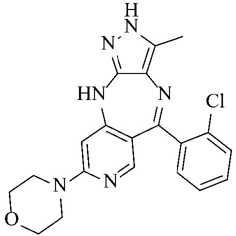 or a pharmaceutically acceptable salt, an isomer, or a crystal form thereof; the (b) novel hormone therapy drug is enzalutamide, apalutamide, darolutamide, bicalutamide, rezvilutamide, abiraterone, or abiraterone acetate; for example, the (b) novel hormone therapy drug is enzalutamide, or abiraterone or abiraterone acetate.

7. A kit, comprising the pharmaceutical composition according to any one of claims 2-6, and instructions for administration.

8. Use of the pharmaceutical combination according to any one of claims 1 and 3-6, or the pharmaceutical composition according to any one of claims 2-6, or the kit according to claim 7 in the manufacture of a medicament for preventing and/or treating prostate cancer.

9. The use according to claim 8, wherein the prostate cancer is:
castration-resistant prostate cancer or castration-sensitive prostate cancer; preferably, metastatic castration-resistant prostate cancer, non-metastatic castration-resistant prostate cancer, or metastatic castration-sensitive prostate cancer, high-risk metastatic hormone therapy-sensitive prostate cancer, non-metastatic castration-sensitive prostate cancer with biochemical recurrence and a high risk of metastasis, or
prostate cancer that has previously failed standard treatment, or prostate cancer that has not previously received standard treatment; preferably, prostate cancer that has previously failed treatment with an androgen synthesis inhibitor and/or an androgen receptor inhibitor; preferably, prostate cancer that has previously failed treatment with enzalutamide, apalutamide, darolutamide, bicalutamide, rezvilutamide, and/or abiraterone acetate.

10. The pharmaceutical combination according to any one of claims 1 and 3-6, or the pharmaceutical composition according to any one of claims 2-6, or the kit according to claim 7, for use in a medicament.

11. The pharmaceutical combination according to any one of claims 1 and 3-6, or the pharmaceutical composition according to any one of claims 2-6, or the kit according to claim 7, for use in preventing and/or treating prostate cancer, wherein preferably, the prostate cancer is:
castration-resistant prostate cancer or castration-sensitive prostate cancer; preferably, metastatic castration-resistant prostate cancer, non-metastatic castration-resistant prostate cancer, or metastatic castration-sensitive prostate cancer, high-risk metastatic hormone therapy-sensitive prostate cancer, non-metastatic castration-sensitive prostate cancer with biochemical recurrence and a high risk of metastasis, or
prostate cancer that has previously failed standard treatment, or prostate cancer that has not previously received standard treatment; preferably, prostate cancer that has previously failed treatment with an androgen synthesis inhibitor and/or an androgen receptor inhibitor; preferably, prostate cancer that has previously failed treatment with enzalutamide, apalutamide, darolutamide, bicalutamide, rezvilutamide, and/or abiraterone acetate.

12. A method for preventing and/or treating prostate cancer, comprising administering to a patient in need thereof a therapeutically effective amount of the pharmaceutical combination according to any one of claims 1 and 3-6, or the pharmaceutical composition according to any one of claims 2-6, or the kit according to claim 7,
wherein preferably, the prostate cancer is:
castration-resistant prostate cancer or castration-sensitive prostate cancer; preferably, metastatic castration-resistant prostate cancer, non-metastatic castration-resistant prostate cancer, or metastatic castration-sensitive prostate cancer, high-risk metastatic hormone therapy-sensitive prostate cancer, non-metastatic castration-sensitive prostate cancer with biochemical recurrence and a high risk of metastasis, or
prostate cancer that has previously failed standard treatment, or prostate cancer that has not previously received standard treatment; preferably, prostate cancer that has previously failed treatment with an androgen synthesis inhibitor and/or an androgen receptor inhibitor; preferably, prostate cancer that has previously failed treatment with enzalutamide, apalutamide, darolutamide, bicalutamide, rezvilutamide, and/or abiraterone or abiraterone acetate.
